(19) Europäisches Patentamt
European Patent Office
Office européen des brevets

(11) EP 3 328 944 B1

(12) EUROPEAN PATENT SPECIFICATION

(45) Date of publication and mention of the grant of the patent:
**06.09.2023 Bulletin 2023/36**

(21) Application number: **16745122.8**

(22) Date of filing: **29.07.2016**

(51) International Patent Classification (IPC):
***C09C 1/02*** *(2006.01)* ***C08K 3/26*** *(2006.01)*
***C08K 7/08*** *(2006.01)*

(52) Cooperative Patent Classification (CPC):
**C09D 7/62; C01F 11/181; C08K 9/04; C09C 1/021; C09C 1/024; C09D 1/00; C09D 7/66; C09D 7/70;**
C01P 2004/03; C01P 2004/16; C01P 2004/17; C01P 2004/45; C01P 2004/50; C01P 2004/64; C01P 2006/12; (Cont.)

(86) International application number:
**PCT/EP2016/068256**

(87) International publication number:
**WO 2017/017281 (02.02.2017 Gazette 2017/05)**

(54) **PRECIPITATED CALCIUM CARBONATE, A METHOD FOR ITS MANUFACTURE AND USES THEREOF**

AUSGEFÄLLTES CALCIUMCARBONAT, VERFAHREN ZU DESSEN HERSTELLUNG UND VERWENDUNGEN DAVON

CARBONATE DE CALCIUM PRÉCIPITÉ, SON PROCÉDÉ DE FABRICATION ET SES UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.07.2015 EP 15179176**

(43) Date of publication of application:
**06.06.2018 Bulletin 2018/23**

(60) Divisional application:
**23184880.5 / 4 230 691**

(73) Proprietor: **IMERTECH SAS**
**75015 Paris (FR)**

(72) Inventors:
- **JAKOB, Alexandra**
**31300 Toulouse (FR)**
- **SY, Didier**
**24460 Négrondes (FR)**
- **RABOTEAU, Cedric**
**13270 Fos sur Mer (FR)**

(74) Representative: **Haseltine Lake Kempner LLP**
**One Portwall Square**
**Portwall Lane**
**Bristol BS1 6BH (GB)**

(56) References cited:
**WO-A1-2005/047372**
**WO-A1-2006/134080**
**WO-A2-2007/014878**
**US-A1- 2004 166 047**
**US-A1- 2010 041 809**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

(52) Cooperative Patent Classification (CPC): (Cont.)
C08K 2003/265; C08K 2201/006; C08K 2201/011

## Description

The present invention concerns precipitated calcium carbonate particles being at least partially in the form of nanofibers or nanochain like agglomerates, wherein the particles are at least partially coated with at least one coating agent, use of such precipitated calcium carbonate particles and a process for the manufacture of such precipitated calcium carbonate particles.

Precipitated calcium carbonate particles are used in a variety of materials, such as plastics and paints, often to replace $TiO_2$ as pigment, generally to enforce the materials, modulate the characteristics of the materials, or to lower production cost. In some cases, it is desirable to provide precipitated calcium carbonate particles which exhibit certain opaque properties and/or high light scattering coefficient as additive in materials such as paints, papers and paper coatings, as described in WO 2013/050495. Coated precipitated calcium carbonate particles can exhibit good properties when used as fillers in polymers and polymer compositions, for example in plastisols, enhancing the mechanical properties of the materials. Coating of precipitated calcium carbonate particles with coatings helps to increase the oleophilicity of the precipitated calcium carbonate particles, thus improving the dispersibility of the fillers in the polymers or polymer compositions, as described in WO 2006/051087.

It has now been found that precipitated calcium carbonate particles being at least partially in the form of nanofibers or nanochain like agglomerates, wherein the particles are at least partially coated with at least one coating agent, exhibit surprisingly improved characteristics when used as additives in materials, in particular polymeric materials, specifically in plastisols. Exceptionally good rheological properties, such as high yield values, can be achieved by such particles in polymeric materials, in particular in plastisol compositions.

Preferred rheological properties in plastisols are for example a high viscosity at low shear such that the plastisol does not sag once applied. A low viscosity at high shear is desirable so that the plastisols can be applied by spraying. The rheological properties are suitable determined, for example, by their Yield Value, which should be sufficiently high. Yield Value can be defined as the stress at which a material begins to deform plastically. Further, good adhesion to a wide variety of substrates, for example concrete, steel, glass, fabrics or plastics, is aimed for. The additive must show a good dispersion of all particles to ensure homogeneity and equal properties, smooth, repeatable processes and to avoid clogging of application apparatus, such as spray nozzles. The precipitated calcium carbonate particles according to the present invention display significantly improved rheological properties, in particular in plastisols, excellent dispersion characteristics and polymeric compositions comprising the precipitated calcium carbonate particles have a very good stability, manufacturing and product properties.

Thus, the present invention concerns precipitated calcium carbonate particles as defined in claim 1. The invention further relates to a process for the preparation of precipitated calcium carbonate particles as defined in claim 8.

The invention further relates to the use of precipitated calcium carbonate particles according to the invention as additive, for example filler, in paints, plastisols, sealants, paper, food products, nutritional products, pharmaceutical products, inks, lacquers and polymers, in particular in plastisols. Paints, plastisols, sealants, papers, food products, nutritional products, pharmaceutical products, inks, lacquers and polymers and polymer compositions, in particular plastisols, comprising precipitated calcium carbonate particles according to the present invention are also subject of the invention.

It is understood that the citation of a singular also intends to include the plural in the present invention; for example, "coating agent" also intends to designate the plural "coating agents".

It is further understood that the word "comprises" in the present invention includes the term "consists of".

The precipitated calcium carbonate particles according to the present invention are at least partially in the form of nanofibers or nanochain like agglomerates. These can be described as constituted by at least two interconnected primary particles. These nanofibers then aggregate themselves in a organized way to form microshells.

The term "particle" is understood to mean a physically and chemically autonomous entity. The term "primary particle" refers to the elementary particles of precipitated calcium carbonate.

In the terms "nanofibers" and "nanochain like agglomerate", the prefix "nano" means that the nanofibers or nanochain like agglomerates have a characteristic dimension at the nanoscale, in particular a characteristic dimension which is, on average, less than 100 nm. In nanofibers or nanochain like agglomerates, said characteristic dimension is the average diameter.

The term "nanofiber" intends to denote an elongated entity having a characteristic dimension, i.e. average diameter, less than 100 nm. The term "nanochain like agglomerate" intends to denote an elongated entity having a characteristic dimension, i.e. average diameter, less than 100 nm. Nanofibers mainly differ from nanochain like agglomerates in that the individual primary particles cannot be distinguished anymore and form nanofibers which appear to be homogeneous and even, for example on electron microscopy photographs, whatever the magnification. In nanochain like agglomerates, the primary particles retain their individuality and remain visible, for example on electron microscopy. Nanochain like agglomerates can also be named "nanorosaries". An essential feature of the present invention resides in the fact that at least part of the precipitated calcium carbonate particles are in the form of nanofibers or nanochain

like agglomerates, such nanofibers or nanochain like agglomerates, which can be described as being constituted by at least two interconnected primary particles and therefore having an elongated morphology. In the present invention, the term "at least partially present in the form of nanofibers or nanochain like agglomerates" denotes that the precipitated calcium carbonate particles are generally present in the form of nanofibers or nanochain like agglomerates in an amount of at least 1 % by weight of the calcium carbonate particles. Often, precipitated calcium carbonate particles are present in the form of nanofibers or nanochain like agglomerates in an amount of at least 8 % by weight of the calcium carbonate particles. In the present invention, precipitated calcium carbonate particles are typically present in the form of nanofibers or nanochain like agglomerates in an amount of at least 10 % by weight of the calcium carbonate particles, more preferably in an amount of at least 15 % by weight of the calcium carbonate. The amount of nanofibers or nanochain like agglomerates has been evaluated relying on SEM (Scanning Electron Microscopy) or TEM (Transmission Electron Microscopy) image analysis. The obtained values correspond to the number of elementary particles that belongs to the nanofibers in respect to the total number of elementary nanoparticles, the measurement being performed in areas of acceptable resolution. It is preferred to determine the amount in a homogenized sample. For the determination using SEM, the calcium carbonate particles are metallized: The samples are directly placed on a graphite tape, then metallized with platinum for 1 minute under a vacuum of $10^{-1}$ Pa with a beam intensity of 6 mA. A Hitachi S-4800 SEM was used for the measurements.

According to the invention, the primary particles are preferably in the form of calcite crystals. The primary particles may be present in a huge variety of shapes, the most common being pseudo-spherical or cubic morphology. Scalenohedral primary particles can also be present when the primary particle is of the calcite type. In another aspect, the primary particles are in the form of aragonite crystals, which often display a needle-like morphology. Mixtures of the foregoing morphologies are also possible.

The nanofibers or nanochain like agglomerates of the precipitated calcium carbonate are further aggregated to form microshells. Microshells may be composed of tens to hundreds of nanofibers or nanochain like agglomerates. In such a case, the nanofibers or nanochain like agglomerates are usually visible at least on the inner part of the microshell like agglomerates.

According to one embodiment of the present invention, precipitated calcium carbonate particles which are at least partially present in the form of nanofibers or nanochain like agglomerates have an average specific surface area of equal to or lower than 60 $m^2g^{-1}$, preferably equal to or lower than 50 $m^2g^{-1}$ (measured by the BET (<u>B</u>runauer-<u>E</u>mmett-<u>T</u>eller) technique described in ISO 9277). Generally, this value applies to the agglomerates before the coating step as well as after the coating step. In this embodiment, the specific surface of the calcium carbonate particles which are at least partially present in the form of nanofibers or nanochain like agglomerates before coating is preferably equal to or higher than 10 $m^2g^{-1}$, more preferably equal to or higher than 15 $m^2g^{-1}$, and most preferably equal to or higher than 20 $m^2g^{-1}$. Also, the specific surface of the calcium carbonate particles before coating is preferably equal to or lower than 60 $m^2g^{-1}$, more preferably equal to or lower than 50 $m^2g^{-1}$, and most preferably equal to or lower than 45 $m^2g^{-1}$. In particular, the specific surface of the calcium carbonate particles before coating is within the range of from 10 to 60 $m^2g^{-1}$, more preferably from 10 to 50 $m^2g^{-1}$, most preferably from 15 to 50 $m^2g^{-1}$ and in particular from 20 to 45 $m^2g^{-1}$. For the purpose of the specification, the specific surface of a precipitated calcium carbonate particle is defined as the surface area which is determined according to the BET-method (adsorption isotherm of nitrogen according to Brunauer-Emmett-Teller (BET)). According to the BET-method the surface of a powder can be calculated from the $N_2$-isotherm, which is observed at the boiling point of liquid nitrogen. For details, it can be referred to the ISO 9277 norm (1995-05-15).

The precipitated calcium particles according to the present invention are at least partially coated with at least one coating agent. In one aspect, only one coating agent is present on the particles. According to the present invention, the at least one coating agent is selected from the group consisting of sulfur organic compounds, fatty acids and the salts thereof.

Generally, the at least one coating agent is comprised in an, if applicable combined, amount of from 0.01 wt% to 15 wt%. Generally, the amount of the at least one coating agent which is comprised in the particles according to the present invention, is equal to or more than 0.01 wt%, preferably equal to or higher than 0.1 wt% and more preferably equal to or higher than 0.2 wt%. Often, the amount of the at least one coating agent which is comprised in the particles according to the present invention, is equal to or less than 15 wt%, preferably equal to or lower than 10 wt% and more preferably equal to or lower than 7 wt%.

In a preferred embodiment of the present invention the coating agent comprises, or consists of, at least one fatty acid, optionally substituted with a hydroxy group. Suitable fatty acids are linear or branched aliphatic carboxylic acids, preferably having 8 to 22 carbon atoms, more preferably 12 to 18 and in particular 16 to 18 carbon atoms, and the salts thereof. The aliphatic chains of the fatty acids may be saturated or unsaturated. Saturated aliphatic fatty acids are preferred. Preferably, the coating agent comprises stearic acid or its salts. Stearic acid is more preferred. Especially preferred is a hydroxy fatty acid or a salt thereof, wherein hydroxy stearic acid is most preferred. Most preferably, the stearic acid bears the hydroxy group in 12-position ($CH_3$-$(CH_2)_4$-$CH_2$-CHOH-$CH_2$-$(CH_2)_8$-$CH_2$- COOH).

Advantageously, the precipitated calcium carbonate particles which are at least partially present in the form of nanofibers or nanochain like agglomerates are treated, either with the fatty acid or hydroxy fatty acid itselves, or with

the alkali metal or ammonium salt thereof. To this end, the hydroxy fatty acid often is used as water-based emulsion comprising sodium hydroxide and/or ammonia. Usually, for ease of handling and to ensure the homogeneity of the ultimate product, aqueous solutions or suspensions of a salt of the fatty acid or hydroxy fatty acid are utilized as the means for effecting the subject surface treatment. When a fatty acid, or its salt, is used as coating agent, the added content of the fatty acid is usually equal to or larger than 0.01 wt%, calculated on the basis of the uncoated particles, preferably equal to or larger than 0.1 wt%, and more preferably equal to or larger than 1 wt%. Further, the added content of the fatty acid or its salt usually is equal to or less than 10 wt%, calculated on the basis of the uncoated particles, preferably equal to or less than 7 wt%, and more preferably equal to or less than 4 wt%. In particular, the preferred content range for hydroxystearin is from 0.1 to 7 wt%, and more preferably from 1 to 4 wt%.

In another preferred embodiment, the precipitated calcium carbonate particles are at least partially coated with at least one sulfur organic compound. Suitable sulfur organic compounds are, for example, an organic sulfonic acid, its ester and/or salt alkylsulfonic acids, alkylsulfosuccinates, alkylsulfates and the salts thereof, their esters and/or salts. In particular, organic sulfonic acids can be alkylsulfonic acids, arylsulfonic acids, alkylarylsulfonic acids, the esters and salts thereof.

For the purpose of the specification, the term "aryl" denotes an aromatic mono- or bicyclic hydrocarbon comprising 6 to 10 carbon atoms, such as phenyl and naphthyl, optionally substituted with at least one halogen atom.

For the purpose of the specification, the term "alkylaryl" denotes an "alkyl" residue as defined above covalently linked to an "aryl"-residue as defined above, such as $-CH_2C_6H_5$, $-CH_2CH_2C_6H_5$, $-CH_2CH_2CH_2C_6H_5$, and the like.

In a preferred embodiment the coating agent comprises a compound represented by general formula (I-A) or (I-B)

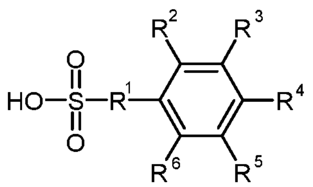

(I-A)

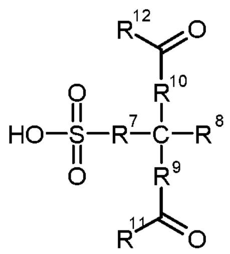

(I-B)

wherein

$R^1$, $R^7$, $R^9$ and $R^{10}$ are independently a single bond, -O-, $-C_1$-$C_{18}$-alkylene- or - $C_2$-$C_{18}$-alkenylene- (wherein in the alkylene- or in the alkenylene-chain optionally 1, 2 or 3 $-CH_2-$ groups may be replaced by -O-);
$R^2$, $R^3$, $R^4$, $R^5$ and $R^6$ are independently -H, $-C_1$-$C_{18}$-alkyl (wherein in the alkyl-chain optionally 1, 2 or 3 $-CH_2-$ groups may be replaced by -O-), -OH, -F, - Cl, -CN, $-CO_2H$, $-CO-C_1$-$C_6$-alkyl, $-CO_2-C_1$-$C_6$-alkyl, $-O-CO-C_1$-$C_6$-alkyl, - $NO_2$, $-NH_2$, $-NH-C_1$-$C_6$-alkyl or $-N(C_1$-$C_6$-alkyl$)_2$; $R^8$ is -H or $-C_1$-$C_6$-alkyl; and
$R^{11}$ and $R^{12}$ are independently -H, $-C_1$-$C_{18}$-alkyl (wherein in the alkyl-chain optionally 1, 2 or 3 $-CH_2-$ groups may be replaced by -O-), - $NH_2$, $-NH-C_1$-$C_6$-alkyl or $-N(C_1$-$C_6$-alkyl$)_2$.

For the purpose of the specification, the term "alkylene" denotes bivalent linear or branched alkylene chains, such as $-CH_2-$, $-CH_2CH_2-$, $-CH_2CH_2CH_2-$, $-CH_2CH_2CH_2CH_2-$, $-CH(CH_3)-$, $-CH_2CH(CH_3)-$, $-CH_2CH_2CH(CH_3)-$, $-CH_2CH(CH_3)CH_2-$, and the like.

For the purpose of the specification, the term "alkenylene" denotes bivalent linear or branched alkenylene chains, such as -CH=CH-, $-CH_2CH$=CH-, $-CH_2CH_2CH$=CH-, -CH=C$(CH_3)$-, $-CH_2CH$=C$(CH_3)$-, -CH=C$(CH_3)CH_2$-, and the like.

Preferably, in the compound represented by general formula (I-A) or (I-B) $R^1$, $R^7$, $R^9$ and $R^{10}$ are independently a single bond or $-C_1$-$C_6$-alkylene- (wherein in the alkylene-chain optionally 1 or 2 $-CH_2-$ groups may be replaced by -O-); $R^2$, $R^3$, $R^4$, $R^5$ and $R^6$ are independently -H or $-C_1$-$C_{18}$-alkyl (wherein in the alkyl-chain optionally 1, 2 or 3 $-CH_2-$ groups may be replaced by -O-); $R^8$ is -H or $-C_1$-$C_6$-alkyl; and
$R^{11}$ and $R^{12}$ are independently $-C_1$-$C_{12}$-alkyl (wherein in the alkyl-chain optionally 1, 2 or 3 $-CH_2-$ groups may be replaced by -O-).

More preferably, in the compound represented by general formula (I-A) or (I-B)

$R^1$ and $R^7$ are a single bond;
$R^2$, $R^3$, $R^5$ and $R^6$ are -H
$R^4$ is -H or $-C_1$-$C_6$-alkyl;

$R^8$ is -H;
$R^9$ and $R^{10}$ are independently a single bond or $-CH_2-$; and
$R^{11}$ and $R^{12}$ are independently $-C_1-C_{12}$-alkoxy.

For the purpose of the specification, the term "alkoxy" denotes an "alkyl" residue as defined above covalently linked to an oxygen atom, such as - $OCH_3$, $-OCH_2CH_3$, and the like.

In a most preferred embodiment, the at least one coating agent is a salt, preferably a sodium salt, of alkylbenzenesulfonic acid (alkylbenzenesulfonate). The preferred alkylbenzenesulfonate, which also corresponds to formula (I-A), is constituted of 12-15 wt% of a toluene sulfonic acid moiety, with R1 being a covalent bond, and one of $R^2$ to $R^6$ being $-CH_3$, and the remaining residues $R^2$ to $R^6$ being optionally branched alkyl. Preferably, the optionally branched alkyl chains are distributed in the following way:

- C9: < 1 wt. %
- C10: 8- 16 wt. %
- C11: 26 - 38 wt. %
- C12: 26 - 38 wt. %
- C13: 15 - 27 wt. %
- C14: < 1 wt. %

When a sulfur organic compound, or its salt, is used as coating agent, the added content of the sulfur organic compound is usually equal to or larger than 0.01 wt%, calculated on the basis of the uncoated particles, preferably equal to or larger than 0.05 wt%, and more preferably equal to or larger than 0.1 wt%. Further, added content of the fatty acid or its salt usually is equal to or less than 10 wt%, calculated on the basis of the uncoated particles, preferably equal to or less than 7 wt%, and more preferably equal to or less than 4 wt%. In particular, the preferred content range for alkylbenzenesulfonic acids or their salts is from 0.01 to 2 wt%, and more preferably from 0.05 to 0.5 wt%.

When a sulfur organic compound, or its salt, is used as coating agent, it is usually dissolved or suspended in an aqueous system. Such an aqueous system can contain, apart from water, other additives, such as salts (for example sodium sulfate and/or sodium chloride), and auxiliary additives such as an oil. Usually the content of such additional additives and auxiliary additives in such a suspension or solution does not exceed 9 wt%.

The precipitated calcium carbonate further comprises at least one crystallization controller, wherein the crystallization controller is polyacrylic acid.

Generally, the molecular weight of the polyacrylic acid is from 500 and 15 000 g/mol. The molecular weight usually is equal to or lager than 500 g/mol, preferably equal to or larger than 700 g/mol and especially preferably equal to or larger than 1000 g/mol. Generally, the molecular weight is equal to or less than 15 000 g/mol, preferably equal to or larger than 10 000 g/mol and especially preferably equal to or larger than 5000 g/mol. Molecular weights of from 1000 to 3500 g/mol are particularly preferred.

Polymeric compositions, in particular plastisol compositions, comprising the precipitated calcium carbonate particles which are at least partially present in the form of nanofibers or nanochain like agglomerates which are at least partially coated with at least one coating agent, as filler generally display an increased yield stress, and thus, improved rheological properties. Generally, such compositions display a yield stress value of from 250 to 600 Pa, measured according to the measurement method described below. Generally, the yield stress value of the compositions comprising the coated particles is equal to or higher than 250 Pa, preferably equal to or higher than 255 Pa and most preferably equal to or higher than 260 Pa. Often, the yield stress value of the compositions comprising the coated particles is equal to or lower than 600 Pa, preferably equal to or lower than 580 Pa and most preferably equal to or lower than 550 Pa. A range of from 280 to 500 Pa is particularly preferred.

The Yield Stress Value is determined using a standardized polymer composition. The polymer composition is defined as follows:

Table 1:

| *formulation* | *Wt%* |
|---|---|
| Plasticizer | 35 |
| Polymer | 25 |
| Solvent | 4 |
| Standard Filler (GCC) | 16 |
| Tested PCC coated particles | 16 |

(continued)

| formulation | Wt% |
|---|---|
| Other additives | 4 |

For determining the Yield Stress Value of a polymer composition comprising the precipitated calcium carbonate particles according to the invention, the following procedure is applied:
The PCC samples are stored for minimum one night at 50% relative humidity and 23°C. The mixer used for preparation of the polymer composition is a Dispermat (VMA).

All liquids are weighed and mixing is started at 300 rpm. Polymer powder is added gradually under mixing, mixing can be increased if necessary to a maximum of 800 rpm. Other ingredients are added at a speed which can be raised, if necessary, to a maximum of 800 rpm. After finalized addition, the polymer composition is mixed for 10 minutes at 800 rpm. The yield value of the composition is then determined according to the following method, using a rheometer Kinexus Pro (Malvern) with a concentric cylinder C14.

Interval 1: no shear rate (waiting time) for 300 s at 23°C
Interval 2: increase of shear rate from 0 to 131 $s^{-1}$ in 120s (20 pts)
Interval 3: shear rate at 131 $s^{-1}$ for 180 s (5 pts)
Interval 4: decrease of shear rate from 131 to 0 $s^{-1}$ in 120 s (20 pts)
The shear stress is plotted vs. shear rate.

The Bingham model is applied on the downward curve (interval 4) from 20 to 131 $s^{-1}$. It allows to obtain the yield value, the Bingham viscosity and the correlation coefficient.

According to a further embodiment, the precipitated calcium carbonate has an average primary particle size (dp) of from 15 to 100 nm. Generally, the average primary particle size (dp) of the precipitated calcium carbonate before coating is equal to or more than 15 nm, more preferably equal to or more than 20 nm and even more preferably equal to or more than 25 nm. Often, the average primary particle size (dp) of the precipitated calcium carbonate before coating is equal to or lower than 100 nm, more preferably equal to or lower than 80 nm and even more preferably equal to or lower than 65 nm. A range of from 25 to 60 nm is particularly preferred. Generally, the values apply to the particles before and after coating. The average primary particle size (dp) is typically measured by permeability, which is described below:
dp is determined by permeability measured according to a method derived from BS 4359-2. The basis of this method is the measurement of the air permeability of a pellet, which is analogous to the "Blaine" or the "Lea & Nurse method". The calculation of the dp derives from the Carman & Malherbe formula :
with

$$q \times L = \frac{1.05\varepsilon^2}{(1-\varepsilon)^2} ds^2 + \frac{2.88\varepsilon^2}{1-\varepsilon} ds$$

$$\varepsilon = 1 - \frac{W}{A \times L \times D}$$

It can be shown that the mean particle diameter ds which is determined according to the Carman & Malherbe formula is not absolutely independent from the porosity of the pellet. Consequently, a correction was brought considering the reference porosity $\varepsilon$ = 0.45 and the dp was calculated according to the formula :

$$dp = ds \times e^{-3.2(\varepsilon - 0,45)}$$

Definitions and unities are as follows :

q = volumetric rate of air flow passed through the PCC pellet ($cm^3/g$),
$\varepsilon$ = porosity,
W = weight of PCC,

L = thickness of the pellet,
D = density of PCC ($g/cm^3$),
A = area of the cross section of the pellet ($cm^2$),
ds = mean particle diameter according to Carman & Malherbe ($\mu m$), and
dp = mean particle diameter according to Solvay ($\mu m$).

According to a further embodiment, the precipitated calcium carbonate before coating has and an aggregate median size D50 of from 0.6 $\mu$m to 4 $\mu$m. Generally, the aggregate median size D50 of the precipitated calcium carbonate before coating is equal to or more than 0.11 $\mu$m, more preferably equal to or more than 0.2 $\mu$m and even more preferably equal to or more than 0.3 $\mu$m. Often, the aggregate median size D50 of the precipitated calcium carbonate before coating is equal to or lower than 10 $\mu$m, more preferably equal to or lower than 6 $\mu$m and even more preferably equal to or lower than 4 $\mu$m. A range of from 0.6 to 4 $\mu$m is particularly preferred. D50 is measured on the basis of French standard ISO 13317-3, "Particle size sedimentation analysis of a powder by variable height gravity in a liquid. Method by X-ray absorption measurement", in which the general method and the apparatus (Sedigraph) are described. Preparation of the sample influencing the results of the measurement, such preparation method is described as follows:
Preparation of the samples for D50 measurement: 2.7 g of precipitated calcium carbonate are introduced into 50 ml of Na-hexametaphosphate (2 g/L) and the solution is treated by magnetical stirring and ultrasound. For the measurements, a Sedigraph 5100® is equipped with an automatic sampler: as Mastertech 51® from Micromeritics is used. The measurement is performed between 0.1 $\mu$m (starting diameter) and 100 $\mu$m (ending diameter). The D50 measurement according to sedimentation method is measured on uncoated calcium carbonate particles.
In a further embodiment, the at least partially coated precipitated calcium carbonate has a loss on drying (LOD) value of equal to or lower than 15 g/kg at 105°C. Generally, the LOD value of the at least partially coated calcium carbonate particles is equal to or more than 0.5 g/kg, preferably equal to or more than 1 g/kg and more preferably equal to or more than 2 g/kg. Often, the LOD value of the at least partially coated calcium carbonate particles is equal to or lower than 15 g/kg, preferably equal to or lower than 12 g/kg and more preferably equal to or lower than 10 g/kg. In a preferred aspect, the LOD value of the at least partially coated calcium carbonate particles is from 4 to 10 g/kg. The LOD is determined according to the following procedure:
Into a weighing bottle, which is pre-dried at 105°C, about 10 g of precipitated calcium carbonate particles are weighed to the nearest 0.1 mg and heated at about 105°C for 3 hours. The bottles are cooled to approximately 22°C in a desiccator, and weighed to the nearest 0.1 mg. Loss on drying is calculated as g/kg = 1000 * (M1-M2)/M1, wherein M1 is the mass of the particles before drying in g, and M2 the mass of the particles after drying in g. The result is given in g/kg with 1 decimal.
The invention also relates to a process for the preparation of precipitated calcium carbonate particles as defined in claim 8.
Generally, the suspension comprising calcium hydroxide and water, which is also denoted as milk of lime (MoL), from which the calcium carbonate particles are precipitated is formed by the reaction of CaO with water or an aqueous medium to form a suspension of $Ca(OH)_2$. Usually, the MoL ($Ca(OH)_2$ in aqueous medium) concentration is from 2 to 20 wt%. In a preferred aspect, the MoL concentration is from 10 to 17 wt%.
The content of calcium carbonate particles in the slurry after precipitation often is equal to or more than 2 wt%, preferably equal to or more than 5 wt% and more preferably equal to or more than 10 wt%. Usually, the content of calcium carbonate particles in the slurry after precipitation is equal to or less than 27 wt%, preferably equal to or less than 25wt% and more preferably equal to or less than 22wt%. The calcium carbonate particles are precipitated by carbonation of the MoL with $CO_2$. Carbon dioxide gas having a concentration of carbon dioxide varying from 3 to 100 % could be used with success. However, it is preferable to use carbon dioxide gas for which the concentration is from 10 to 60 % by volume, especially from 10 to 50 % by volume, and in particular, 23 to 40 % by volume, carbon dioxide gas being diluted with air or other inert gas. Some auxiliary additives might also be further added during or after, preferably after, the carbonation step, such as ascorbic or isoascorbic acid, to reduce yellowness of the resulting calcium carbonate particles.
The precipitation of calcium carbonate particles in step (a) usually is started at a temperature of the MoL which is from 10 to 30°C. Preferably, the temperature of the MoL at the start of the carbonation is from 12-20°C. At the end of carbonation, a temperature of from 15-70°C, in particular of from 55-65°C is maintained. Heating or cooling is applied during the carbonation when necessary to keep the MoL or slurry temperature in the given range.
The slurry of precipitated calcium carbonate obtained by the carbonation often has a final concentration of equal to or more than 3 wt%, preferably equal to or more than 7 wt%, and most preferably of equal to or more than 15 wt%. Generally, the slurry of precipitated calcium carbonate obtained by the carbonation often has a final concentration of equal to or less than 27 wt%, preferably equal to or less than 25 wt%, and most preferably of equal to or less than 22 wt%. A range of from 15 to 22 wt% is most particularly preferred.
Usually, the suspension is stirred during step (a). If required, the suspension is stirred up to 120 minutes after carbonation is stopped to complete precipitation.

In one embodiment of the present invention, the particles are obtained as a suspension in step (a) and are directly contacted with the at least one coating agent without intermediate separation of the particles from the aqueous phase of the suspension. In another embodiment, the particles are separated/recovered from the aqueous phase of the suspension formed in step (a) before being contacted with the at least one coating agent in step (b).

In a preferred embodiment, the suspension of precipitated calcium carbonate obtained by the carbonation of MoL is contacted with the at least one coating agent. Preferably, one coating agent is added in step (b). Often, the at least one coating agent is added to the suspension formed in step (a) dissolved or suspended in an aqueous medium, thus forming an aqueous coating agent composition comprising at least one coating agent. The hydroxystearin coating agent formulation preferably contains also a base, in a preferred aspect a metal hydroxide, preferably sodium hydroxide, or ammonia. In one aspect, the amount of base present is selected such as to fully neutralize the hydroxy stearin. In one embodiment, the at least coating agent or coating agent formulation is added to the precipitated calcium carbonate particles, preferably, the precipitated calcium particle suspension obtained in step (a), in step (b) at a defined temperature. The temperature is dependent on the nature of the at least one coating agent, and generally is in the range of from 10 to 95°C. In one embodiment, when the coating agent formulation is a hydroxystearin formulation, the precipitated calcium carbonate suspension often has a temperature of equal to or more than 50°C, more preferably equal to or more than 60°C, and even more preferably equal to or more than 70°C. Generally, when the coating agent formulation is a hydroxystearin formulation, the precipitated calcium carbonate suspension often has a temperature of equal to or less than 90°C, more preferably equal to or less than 85°C, and even more preferably equal to or less than 80°C. The most preferred temperature range at which the hydroxystearin coating formulation is added to the suspension of precipitated calcium carbonate is from 70 to 80°C. In another embodiment, when the coating agent formulation is a metal, in particular sodium, alkylbenzenesulfonate formulation, the precipitated calcium carbonate suspension often has a temperature of equal to or more than 10°C, more preferably equal to or more than 20°C, and even more preferably equal to or more than 30°C. Generally, when the coating agent formulation is a metal, in particular sodium, alkylbenzenesulfonate formulation, the precipitated calcium carbonate suspension often has a temperature of equal to or less than 70°C, more preferably equal to or less than 65°C, and even more preferably equal to or less than 60°C. The most preferred temperature range at which the metal, in particular sodium, alkylbenzenesulfonate coating formulation is added to the suspension of precipitated calcium carbonate is from 45 to 55°C. The temperature of the precipitated calcium carbonate suspension is maintained during the addition of the at least one coating agent or coating agent formulation.

After the at least one coating agent is added to the precipitated calcium carbonate suspension in step (b), the mixture is stirred for a time ranging from 5 to 120 minutes, preferably from 5 to 30 minutes. Often, the temperature given for the addition of the at least one coating agent or coating agent formulation is maintained during stirring.

In another embodiment, the precipitated calcium carbonate is separated from the aqueous phase formed in step (a) by appropriate techniques, such as filtering or spinning and optionally dried, for example by heat application. The wet, separated or optionally dried precipitated calcium carbonate particles are then contacted with the at least one coating agent or coating agent formulation, which are described above, by appropriate techniques, such as milling or stirring, wherein stirring is preferred.

At least one crystallization controller is present in step (a). Preferably, one crystallization controller is present in step (a). The at least one crystallization controller is added to the milk of lime (MoL) before start of the carbonation. The crystallization controller is polyacrylic acid.

Generally the molecular weight of the polyacrylic acid is from 500 and 15 000 g/mol. The molecular weight usually is equal to or larger than 500 g/mol, preferably equal to or larger than 700 g/mol and especially preferably equal to or larger than 1000 g/mol. Generally the molecular weight is equal to or less than 15 000 g/mol, preferably equal to or larger than 10 000 g/mol and especially preferably equal to or larger than 5000 g/mol. Molecular weights of from 1000 to 3500 g/mol are particularly preferred.

Often, the at least one crystallization controller is added to the milk of lime before carbonation of the milk of lime as an aqueous crystallization controller formulation. In another aspect, the crystallization controller is added to the milk of lime neat. The polyacrylic acid is present in the milk of lime in an amount from 0.1 to 1 wt%. Often, when the at least one crystallization controller is polyacrylic acid, either in its free form or fully or partially neutralized with metal ions such as sodium, which is added to the milk of lime in an aqueous formulation, the amount of polyacrylic acid in the polyacrylic acid crystallization controller formulation is equal to or more than 40 wt%, preferably equal to or more than 45 wt%, and more preferably equal to or more than 50 wt%. Generally, the amount of polyacrylic acid in the polyacrylic acid formulation is equal to or less than 70 wt%, preferably equal to or less than 65 wt%, and more preferably equal to or less than 60 wt%. The most preferred range of polyacrylic acid in the polyacrylic acid formulation to be added in step (a) is from 53-55wt%.

According to the preferred embodiment, the aqueous suspension of precipitated calcium carbonate particles as obtained in step (a) are contacted with the coating agent without being separated from the aqueous phase of the suspension. Generally, after completion of step (b), the suspension comprising the at least partially coated precipitated calcium carbonate particles is submitted to a step (c) in which the at least partially coated precipitated calcium carbonate

particles are separated from the aqueous phase by appropriate techniques, such as filtration or spinning, and optionally one or more washing steps. In a further optional step (d), the wet cake of at least partially coated precipitated calcium carbonate particles is dried according to appropriate drying techniques, for example in an oven or by infrared radiation. In a further optional step (e), the at least partially coated precipitated calcium carbonate particles, are milled to avoid the presence of big agglomerates with a size exceeding 100 $\mu$m. Optional steps (c) to (e) can be combined in either order and number. A succession of steps (c), (d) and (e) is preferred.

The present invention also concerns the use of the at least partially coated precipitated calcium carbonate particles according to the present invention as additive, for example filler, in paints, plastisols, sealants, paper, food products, nutritional products, pharmaceutical products, inks, lacquers and polymers. The use of the at least partially coated precipitated calcium carbonate particles according to the present invention in plastisols is particularly preferred. The plastisols comprising the at least partially coated precipitated calcium carbonate particles according to the present invention exhibit excellent rheological and other advantageous properties.

The present invention also concerns paint, plastisol, sealant, paper, food product, nutritional product, pharmaceutical product, ink, lacquer, polymer and polymer compositions, comprising at least partially coated precipitated calcium carbonate particles according to the present invention. Plastisols comprising the at least partially coated precipitated calcium carbonate particles according to the present invention are particularly preferred.

In a preferred embodiment, the invention concerns a plastisol, which contains

- the coated particles according to the invention;
- at least one polymer, such as polyvinyl chloride (PVC), e.g. Vestolit, Vinolit,
- optionally at least one plasticizer, such as di-octylphtalate (DOP), di-isononylphtalate (DINP), Adipate, Sebacate
- Optionally at least one solvent, such as hydrocarbons (e.g. the solvent may be or comprise a hydrocarbon), derivatives of hydrocarbons (e.g. the solvent may be or comprise a derivative of a hydrocarbon) and/or a dearomatized fluid, such as a dearomatized hydrocarbon fluid
- optionally at least one filler, such as $TiO_2$, $SiO_2$, Natural Calcium Carbonate
- optionally at least one promoter, such as Polyamines, Polyamides
- optionally at least one desiccant, such as CaO and
- optionally further additives.

Preferred embodiments of the compositions according to the invention are summarized here below :

Table 2 :

| [wt.-%] | preferably | more preferably | most preferably |
|---|---|---|---|
| coated particles | 1-50 | 5-30 | 15-25 |
| polymer | 5-75 | 15-50 | 25-35 |
| plasticizer | 10-60 | 20-50 | 30-40 |
| Solvent | 0.5-15 | 1-10 | 3-6 |
| filler | 0-30 | 0-20 | 0-16 |
| promoter | 0-20 | 0-10 | 1-6 |
| desiccant | 0-20 | 0-10 | 1-2 |
| further additives | 0-15 | 0-10 | 0-5 |

The following examples further illustrate the invention but are not to be construed as limiting its scope.

*Example 1 : preparation of precipitated calcium carbonate (PCC)*

A milk of lime with a concentration of 15% wt. $Ca(OH)_2$ and an initial temperature of 17.5°C was enriched with 0.25 wt% polyacrylic acid (about 2500 g/mol). Carbonation of the milk of lime occurred under a 28% $CO_2$ flow (diluted with air) at a flow rate of 15 $m^3$/h. The final carbonation temperature was 37.8°C. A sample of the slurry was filtered, dried in an oven and analyzed with SEM. The sample was found to be constituted of nanochain like agglomerates, combined to form microshell like aggregates. Average diameter and average length were respectively 70 nm and 284 nm. These values are based on the measurement of 10 nanofibers.

*Example 2: coating of precipitated calcium carbonate (PCC) with hydroxystearin*

The slurry obtained in experiment 1 is brought to a temperature of 70°C, and a hydroxystearin emulsion in water is added in an amount corresponding to 30 g/kg of hydroxystearin. The mixture is filtered on a planar filter and is dried in an oven for 36h at 95°C, before milling in an Alpine 160 Z at 14 800 rpm.
Loss on drying for this product at 105°C is 3.4 g/kg; the specific surface area is found to be 22 $m^2/g$.
Fig. 1 shows a SEM micrography of the obtained PCC2.

*Example 3: coating of precipitated calcium carbonate (PCC) with sodium alkylbenzenesulfonate*

The slurry obtained in experiment 1 is brought to a temperature of 50°C, and a sodium alkylbenzenesulfonate emulsion in water is added in an amount corresponding to 10 g/kg of sodium alkylbenzenesulfonate. The mixture is filtered on a planar filter and is dried in an oven for 36h at 95°C, before milling in an Alpine 160 Z at 14 800 rpm.
Loss on drying at 105°C is 7.9 g/kg for this product and specific surface area of 27 $m^2/g$ is found.
Fig. 2 shows a SEM micrography of the obtained PCC3.

*Example 4: application of precipitated calcium carbonate in plastisol formulation*

The surface treated calcium carbonate particles obtained by experiment 2 (PCC2) and 3 (PCC 3) were measured according to the Yield Stress Value test described above.
The comparative example is Socal ® 312, which is an ultrafine PCC coated with 28 g/kg stearin. It displays an LOD value of 4.5 g/kg and a specific surface area of 22 $m^2/g$. Socal ® 312 has a calcite rhombohedral crystal structure, and a cubic particle shape. The particles are not agglomerated in nanochains or nanofibres. Fig. 3 shows a SEM micrography of Socal ® 312.

| PCC | Yield Stress Value |
|---|---|
| Comparative Example Socal ® 312 | 218 |
| PCC 2 | 285 |
| PCC 3 | 300 |

Table 2 :

| [wt.-%] | preferably | more preferably | most preferably |
|---|---|---|---|
| coated particles | 1-50 | 5-30 | 15-25 |
| polymer | 5-75 | 15-50 | 25-35 |
| plasticizer | 10-60 | 20-50 | 30-40 |
| Solvent | 0.5-15 | 1-10 | 3-6 |
| filler | 0-30 | 0-20 | 0-16 |
| promoter | 0-20 | 0-10 | 1-6 |
| desiccant | 0-20 | 0-10 | 1-2 |
| further additives | 0-15 | 0-10 | 0-5 |

The following examples further illustrate the invention but are not to be construed as limiting its scope.

*Example 1 : preparation of precipitated calcium carbonate (PCC)*

A milk of lime with a concentration of 15 % wt. $Ca(OH)_2$ and an initial temperature of 17,5°C was enriched with 0.25 wt% polyacrylic acid (about 2500 g/mol). Carbonation of the milk of lime occurred under a 28 % $CO_2$ flow (diluted with air) at a flow rate of 15 $m^3/h$. The final carbonation temperature was 37.8 °C. A sample of the slurry was filtered, dried in an oven and analyzed with SEM. The sample was found to be constituted of nanochain like agglomerates, combined to form microshell like aggregates. Average diameter and average length were respectively 70 nm and 284

nm. These values are based on the measurement of 10 nanofibers.

*Example 2: coating of precipitated calcium carbonate (PCC) with hydroxystearin*

The slurry obtained in experiment 1 is brought to a temperature of 70°C, and a hydroxystearin emulsion in water is added in an amount corresponding to 30 g/kg of hydroxystearin. The mixture is filtered on a planar filter and is dried in an oven for 36h at 95°C, before milling in an Alpine 160 Z at 14 800 rpm.

Loss on drying for this product at 105°C is 3.4 g/kg; the specific surface area is found to be 22 $m^2/g$.

Fig. 1 shows a SEM micrography of the obtained PCC2.

*Example 3: coating of precipitated calcium carbonate (PCC) with sodium alkylbenzenesulfonate*

The slurry obtained in experiment 1 is brought to a temperature of 50°C, and a sodium alkylbenzenesulfonate emulsion in water is added in an amount corresponding to 10 g/kg of sodium alkylbenzenesulfonate. The mixture is filtered on a planar filter and is dried in an oven for 36h at 95°C, before milling in an Alpine 160 Z at 14 800 rpm.

Loss on drying at 105 °C is 7.9 g/kg for this product and specific surface area of 27 $m^2/g$ is found.

Fig. 2 shows a SEM micrography of the obtained PCC3.

*Example 4: application of precipitated calcium carbonate in plastisol formulation*

The surface treated calcium carbonate particles obtained by experiment 2 (PCC2) and 3 (PCC 3) were measured according to the Yield Stress Value test described above.

The comparative example is Socal ® 312, which is an ultrafine PCC coated with 28 g/kg stearin. It displays an LOD value of 4.5 g/kg and a specific surface area of 22 $m^2/g$. Socal ® 312 has a calcite rhombohedral crystal structure, and a cubic particle shape. The particles are not agglomerated in nanochains or nanofibres. Fig. 3 shows a SEM micrography of Socal ® 312.

| PCC | Yield Stress Value |
|---|---|
| Comparative Example Socal ® 312 | 218 |
| PCC 2 | 285 |
| PCC 3 | 300 |

## Claims

1. Precipitated calcium carbonate particles being at least partially in the form of nanofibers or nanochain like agglomerates, wherein the nanofibers or nanochain like agglomerates are further aggregated to form microshells, and wherein the particles are at least partially coated with at least one coating agent, wherein the precipitated calcium carbonate particles that are at least partially coated with at least one coating agent are formable in a process which comprises a step of contacting precipitated calcium carbonate particles being at least partially in the form of nanofibres or nanochain like agglomerates with at least one coating agent, wherein the nanofibers or nanochain like agglomerates are further aggregated to form microshells wherein the process comprises:

   (a) precipitating calcium carbonate particles from a suspension comprising calcium hydroxide and water, which is termed milk of lime, under conditions providing precipitated calcium carbonate particles being at least partially in the form of the nanofibers or nanochain like agglomerates, wherein the suspension further comprises polyacrylic acid as a crystallization controller, wherein the polyacrylic acid is added before carbonation and wherein the polyacrylic acid is present in the milk of lime in an amount of 0.1 to 1wt%, and
   (b) contacting the particles of calcium carbonate obtained from step (a) with the at least one coating agent.

2. Precipitated calcium carbonate particles according to claim 1, wherein the nanofibers or nanochain like agglomerates have an average specific surface area of equal to or lower than 60 $m^2/g$, preferably equal to or lower than 50 $m^2/g$ before coating (measured by the BET technique described in ISO 9277).

3. The precipitated calcium carbonate according to claim 1 or 2, wherein the at least one coating agent is selected from the group consisting of sulfur organic compounds, fatty acids and the salts thereof, preferably wherein the at

least one coating agent is a fatty acid or salt thereof or an organic sulfonic acid, its ester and/or salt.

**4.** The precipitated calcium carbonate according to any one of claims 1 to 3, wherein the coating agent comprises a compound represented by general formula (I-A) or (I-B)

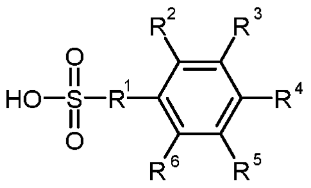

(I-A)

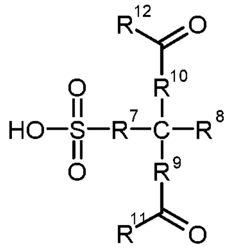

(I-B)

wherein

$R^1$, $R^7$, $R^9$ and $R^{10}$ are independently a single bond, -O-, -$C_1$-$C_{18}$-alkylene- or - $C_2$-$C_{18}$-alkenylene- (wherein in the alkylene- or in the alkenylene-chain optionally 1, 2 or 3 -$CH_2$- groups may be replaced by -O-);
$R^2$, $R^3$, $R^4$, $R^5$ and $R^6$ are independently -H, -$C_1$-$C_{18}$-alkyl (wherein in the alkyl-chain optionally 1, 2 or 3 -$CH_2$- groups may be replaced by -O-), -OH, -F, - Cl, -CN, -$CO_2$H, -CO-$C_1$-$C_6$-alkyl, -$CO_2$-$C_1$-$C_6$-alkyl, -O-CO-$C_1$-$C_6$-alkyl, - $NO_2$, -$NH_2$, -NH-$C_1$-$C_6$-alkyl or -N($C_1$-$C_6$-alkyl)$_2$;
$R^8$ is -H or -$C_1$-$C_6$-alkyl; and
$R^{11}$ and $R^{12}$ are independently -H, -$C_1$-$C_{18}$-alkyl (wherein in the alkyl-chain optionally 1, 2 or 3 -$CH_2$- groups may be replaced by -O-), - $NH_2$, -NH-$C_1$-$C_6$-alkyl or -N($C_1$-$C_6$-alkyl)$_2$.

**5.** The precipitated calcium carbonate according to any one of claims 1 to 4 wherein the at least one coating agent is comprised in an, if applicable combined, amount of from 0.01 wt% to 15 wt%.

**6.** The precipitated calcium carbonate according to any one of claims 1 to 5 wherein the precipitated calcium carbonate before coating has an average primary particle size (dp) of from 15 to 100 nm, and an aggregate median size of from 0.11 μm to 10 μm.

**7.** The precipitated calcium carbonate according to any one of claims 1 to 6 wherein the precipitated calcium carbonate has a loss on drying value of equal to or lower than 15 g/kg at 105°C.

**8.** Process for the preparation of precipitated calcium carbonate particles being at least partially in the form of nanofibres or nanochain like agglomerates, wherein the particles are at least partially coated with at least one coating agent, which comprises a step of contacting precipitated calcium carbonate particles being at least partially in the form of nanofibres or nanochain like agglomerates with at least one coating agent, wherein the nanofibers or nanochain like agglomerates are further aggregated to form microshells, wherein the process comprises:

(a) precipitating calcium carbonate particles from a suspension comprising calcium hydroxide and water under conditions providing precipitated calcium carbonate particles being at least partially in the form of the nanofibers or nanochain like agglomerates, wherein the suspension further comprises polyacrylic acid as a crystallization controller, wherein the polyacrylic acid is added before carbonation and wherein the polyacrylic acid is present in the milk of lime in an amount of 0.1 to 1wt%, and
(b) contacting the particles of calcium carbonate obtained from step (a) with the at least one coating agent.

**9.** The process according to claim 8, wherein the precipitation of calcium carbonate particles in step (a) is started at a temperature of the milk of lime which is from 10 to 30°C.

**10.** The process according to claim 8 or claim 9, wherein the temperature of the milk of lime at the start of carbonation is from 12 to 20°C.

**11.** The process according to claims 8 to 10, wherein at the end of carbonation, a temperature of from 15 to 70°C, preferably from 55 to 65°C is maintained.

**12.** Use of precipitated calcium carbonate particles according to any one of claims 1 to 7 as additive, for example filler, in paints, plastisols, sealants, paper, food products, nutritional products, pharmaceutical products, inks, lacquers and polymers, in particular in plastisols.

**13.** Paint, plastisol, sealant, paper, food product, nutritional product, pharmaceutical product, ink, lacquer, polymer and polymer compositions, in particular plastisol, comprising precipitated calcium carbonate particles according to any one of claims 1 to 7.

**14.** Plastisol comprising precipitated calcium carbonate particles according to any one of claims 1 to 7.

## Patentansprüche

**1.** Ausgefällte Calciumcarbonatpartikel, die zumindest teilweise in der Form von Nanofasern oder nanokettenartigen Agglomeraten vorliegen, wobei die Nanofasern oder nanokettenartigen Agglomerate ferner aggregiert sind, um Mikrohüllen auszubilden, und wobei die Partikel zumindest teilweise mit mindestens einem Beschichtungsmittel beschichtet sind, wobei die ausgefällten Calciumcarbonatpartikel, die zumindest teilweise mit mindestens einem Beschichtungsmittel beschichtet sind, in einem Verfahren formbar sind, welches einen Schritt des Inkontaktbringens ausgefällter Calciumcarbonatpartikel, die zumindest teilweise in der Form von Nanofasern oder nanokettenartigen Agglomeraten vorliegen, mit mindestens einem Beschichtungsmittel umfasst, wobei die Nanofasern oder nanokettenartigen Agglomerate ferner aggregiert werden, um Mikrohüllen auszubilden, wobei das Verfahren Folgendes umfasst:

(a) Ausfällen von Calciumcarbonatpartikeln aus einer Suspension, die Calciumhydroxid und Wasser umfasst, welche als Kalkmilch bezeichnet wird, unter Bedingungen, die dafür sorgen, dass ausgefällte Calciumcarbonatpartikel zumindest teilweise in der Form von Nanofasern oder nanokettenartigen Agglomeraten vorliegen, wobei die Suspension ferner Polyacrylsäure als einen Kristallisationsregler umfasst, wobei die Polyacrylsäure vor der Karbonisierung zugegeben wird und wobei die Polyacrylsäure in der Kalkmilch in einer Menge von 0,1 bis 1 Gew.-% vorhanden ist, und
(b) Inkontaktbringen der aus Schritt (a) erhaltenen Calciumcarbonatpartikel mit dem mindestens einen Beschichtungsmittel.

**2.** Ausgefällte Calciumcarbonatpartikel nach Anspruch 1, wobei die Nanofasern oder nanokettenartigen Agglomerate vor dem Beschichten (gemessen mit der in ISO 9277 beschriebenen BET-Technik) eine durchschnittliche spezifische Oberfläche von gleich oder kleiner als 60 $m^2/g$, vorzugsweise gleich oder kleiner als 50 $m^2/g$, aufweisen.

**3.** Ausgefälltes Calciumcarbonat nach Anspruch 1 oder 2, wobei das mindestens eine Beschichtungsmittel aus der Gruppe bestehend aus schwefelorganischen Verbindungen, Fettsäuren und Salzen davon ausgewählt ist, wobei das mindestens eine Beschichtungsmittel vorzugsweise eine Fettsäure oder ein Salz davon oder eine organische Sulfonsäure, deren Ester und/oder Salz ist.

**4.** Ausgefälltes Calciumcarbonat nach einem der Ansprüche 1 bis 3, wobei das Beschichtungsmittel eine Verbindung umfasst, die durch die allgemeinen Formel (I-A) oder (I-B) dargestellt ist:

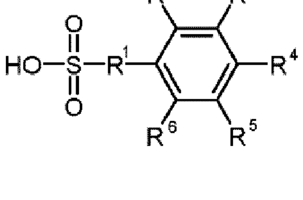

(I-A)    (I-A)

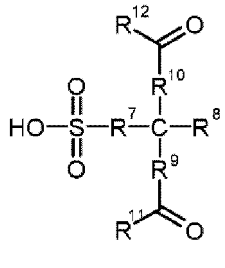

(I-B)    (I-B)

wobei

$R^1$, $R^7$, $R^9$ und $R^{10}$ unabhängig eine Einfachbindung, -O-, $-C_1-C_{18}$-Alkylen- oder $-C_2-C_{18}$-Alkenylen- (wobei in

der Alkylen- oder in der Alkenylen-Kette optional 1, 2 oder 3 $-CH_2$-Gruppen durch -O-ersetzt sein können) sind; $R^2$, $R^3$, $R^4$, $R^5$ und $R^6$ unabhängig -H, $-C_1$-$C_{18}$-Alkyl (wobei in der Alkylkette optional 1, 2 oder 3 $-CH_2$-Gruppen durch -O- ersetzt sein können), -OH, -F, -Cl, -CN, $-CO_2H$, $-CO-C_1$-$C_6$-Alkyl, $-CO_2-C_1$-$C_6$-Alkyl, $-O-CO-C_1$-$C_6$-Alkyl, $-NO_2$, $-NH_2$, $-NH-C_1$-$C_6$-Alkyl oder - $N(C_1$-$C_6$-Alkyl$)_2$ sind;
$R^8$ -H oder $-C_1$-$C_6$-Alkyl ist; und
$R^{11}$ und $R^{12}$ unabhängig -H, $-C_1$-$C_{18}$-Alkyl (wobei in der Alkylkette optional 1, 2 oder 3 $-CH_2$-Gruppen durch -O- ersetzt sein können), $-NH_2$, - $NH-C_1$-$C_6$-Alkyl oder $-N(C_1$-$C_6$-Alkyl$)_2$ sind.

**5.** Ausgefälltes Calciumcarbonat nach einem der Ansprüche 1 bis 4, wobei das mindestens eine Beschichtungsmittel in einer, gegebenenfalls kombinierten, Menge von 0,01 Gew.-% bis 15 Gew.-% enthalten ist.

**6.** Ausgefälltes Calciumcarbonat nach einem der Ansprüche 1 bis 5, wobei das ausgefällte Calciumcarbonat vor dem Beschichten eine durchschnittliche Primärpartikelgröße (dp) von 15 bis 100 nm und eine mittlere Aggregatgröße von 0,11 um bis 10 um aufweist.

**7.** Ausgefälltes Calciumcarbonat nach einem der Ansprüche 1 bis 6, wobei das ausgefällte Calciumcarbonat einen Trocknungsverlustwert von gleich oder kleiner als 15 g/kg bei 105 °C aufweist.

**8.** Verfahren zur Herstellung von ausgefällten Calciumcarbonatpartikeln, die zumindest teilweise in der Form von Nanofasern oder nanokettenartigen Agglomeraten vorliegen, wobei die Partikel zumindest teilweise mit mindestens einem Beschichtungsmittel beschichtet sind, welches einen Schritt des Inkontaktbringens von ausgefällten Calciumcarbonatpartikeln, die zumindest teilweise in der Form von Nanofasern oder nanokettenartigen Agglomeraten vorliegen, mit mindestens einem Beschichtungsmittel umfasst, wobei die Nanofasern oder nanokettenartigen Agglomerate ferner aggregiert werden, um Mikrohüllen auszubilden, wobei das Verfahren Folgendes umfasst:

(a) Ausfällen von Calciumcarbonatpartikeln aus einer Suspension, die Calciumhydroxid und Wasser unter Bedingungen umfasst, die dafür sorgen, dass ausgefällte Calciumcarbonatpartikel zumindest teilweise in der Form von Nanofasern oder nanokettenartigen Agglomeraten vorliegen, wobei die Suspension ferner Polyacrylsäure als einen Kristallisationsregler umfasst, wobei die Polyacrylsäure vor der Karbonisierung zugegeben wird und wobei die Polyacrylsäure in der Kalkmilch in einer Menge von 0,1 bis 1 Gew.-% vorhanden ist, und
(b) Inkontaktbringen der aus Schritt (a) erhaltenen Calciumcarbonatpartikel mit dem mindestens einen Beschichtungsmittel.

**9.** Verfahren nach Anspruch 8, wobei die Ausfällung von Calciumcarbonatpartikeln in Schritt (a) bei einer Temperatur der Kalkmilch begonnen wird, welche zwischen 10 und 30 °C liegt.

**10.** Verfahren nach Anspruch 8 oder Anspruch 9, wobei die Temperatur der Kalkmilch zu Beginn der Karbonisierung 12 bis 20 °C beträgt.

**11.** Verfahren nach den Ansprüchen 8 bis 10, wobei am Ende der Karbonisierung eine Temperatur von 15 bis 70 °C, vorzugsweise von 55 bis 65 °C, aufrechterhalten wird.

**12.** Verwendung von ausgefällten Calciumcarbonatpartikeln nach einem der Ansprüche 1 bis 7 als Zusatzstoff, beispielsweise Füllstoff, in Farben, Plastisolen, Dichtstoffen, Papier, Lebensmitteln, Ernährungsprodukten, pharmazeutischen Produkten, Tinten, Lacken und Polymeren, insbesondere in Plastisolen.

**13.** Farbe, Plastisol, Dichtungsmittel, Papier, Lebensmittelprodukt, Ernährungsprodukt, pharmazeutisches Produkt, Tinte, Lack, Polymer und Polymerzusammensetzungen, insbesondere Plastisol, umfassend ausgefällte Calciumcarbonatpartikel nach einem der Ansprüche 1 bis 7.

**14.** Plastisol, umfassend ausgefällte Calciumcarbonatpartikel nach einem der Ansprüche 1 bis 7.

## Revendications

**1.** Particules de carbonate de calcium précipitées se présentant au moins partiellement sous la forme de nanofibres ou de nanochaînes comme agglomérats, les nanofibres ou les nanochaînes comme agglomérats étant en outre agrégés pour former des microcoques, et les particules étant au moins partiellement recouvertes d’au moins un

agent de revêtement, dans lequel les particules de carbonate de calcium précipitées qui sont au moins partiellement recouvertes d'au moins un agent de revêtement peuvent être formées dans un processus qui comprend une étape de mise en contact de particules de carbonate de calcium précipitées se présentant au moins partiellement sous la forme de nanofibres ou de nanochaînes comme agglomérats avec au moins un agent de revêtement, dans lequel les nanofibres ou nanochaînes comme agglomérats sont en outre agrégés pour former des microcoques, le processus comprenant :

(a) la précipitation des particules de carbonate de calcium à partir d'une suspension comprenant de l'hydroxyde de calcium et de l'eau, qui est appelée eau de chaux, dans des conditions fournissant des particules de carbonate de calcium précipitées se présentant au moins partiellement sous la forme de nanofibres ou de nanochaînes comme agglomérats, la suspension comprenant en outre l'acide polyacrylique comme régulateur de cristallisation, dans lequel l'acide polyacrylique est ajouté avant la carbonatation et dans lequel l'acide polyacrylique est présent dans l'eau de chaux en une quantité de 0,1 à 1 % en poids, et
(b) la mise en contact des particules de carbonate de calcium obtenues à l'étape (a) avec l'au moins un agent de revêtement.

2. Particules de carbonate de calcium précipitées selon la revendication 1, dans lesquelles les nanofibres ou nanochaînes comme agglomérats ont une surface spécifique moyenne égale ou inférieure à 60 $m^2/g$, de préférence égale ou inférieure à 50 $m^2/g$ avant recouvrement (mesurée par la technique BET décrite dans l'ISO 9277) .

3. Carbonate de calcium précipité selon la revendication 1 ou 2, dans lequel l'au moins un agent de revêtement est choisi dans le groupe constitué par les composés organiques soufrés, les acides gras et leurs sels, de préférence dans lequel l'au moins un agent de revêtement est un acide gras ou un sel de celui-ci ou un acide sulfonique organique, son ester et/ou son sel.

4. Carbonate de calcium précipité selon l'une quelconque des revendications 1 à 3, dans lequel l'agent de revêtement comprend un composé représenté par la formule générale (I-A) ou (I-B)

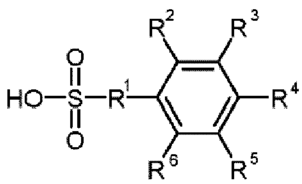

(I-A)

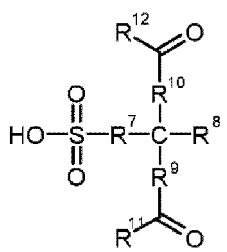

(I-A) -B) (I-B)

dans lequel

$R^1$, $R^7$, $R^9$ et $R^{10}$ sont indépendamment une simple liaison, -O-, - $C_1$-$C_{18}$-alkylène- ou -$C_2$-$C_{18}$-alkénylène- (dans lequel dans l'alkylène- ou dans la chaîne-alkénylène éventuellement 1, 2 ou 3 groupes -$CH_2$- peuvent être remplacés par -O-) ;
$R^2$, $R^3$, $R^4$, $R^5$ et $R^6$ sont indépendamment -H, -$C_1$-$C_{18}$-alkyle (où dans la chaîne alkyle éventuellement 1, 2 ou 3 groupes -$CH_2$-peuvent être remplacés par -O-), -OH, -F, -Cl, -CN, -$CO_2$H, -CO-$C_1$-$C_6$-alkyle, -$CO_2$-$C_1$-$C_6$-alkyle, -O-CO-$C_1$-$C_6$-alkyle, -$NO_2$, -$NH_2$, - NH-$C_1$-$C_6$-alkyle ou -N($C_1$-$C_6$-alkyle)$_2$;
$R^8$ est -H ou -$C_1$-$C_6$-alkyle ; et
$R^{11}$ et $R^{12}$ sont indépendamment -H, -$C_1$-$C_{18}$-alkyle (où dans la chaîne-alkyle éventuellement 1, 2 ou 3 groupes -$CH_2$- peuvent être remplacés par -O-), -$NH_2$, -NH-$C_1$-$C_6$-alkyle ou -N($C_1$-$C_6$-alkyle)$_2$.

5. Carbonate de calcium précipité selon l'une quelconque des revendications 1 à 4, dans lequel l'au moins un agent de revêtement est compris dans une quantité, le cas échéant combinée, de 0,01 % en poids à 15 % en poids.

6. Carbonate de calcium précipité selon l'une quelconque des revendications 1 à 5 dans lequel le carbonate de calcium précipité avant revêtement a une taille moyenne de particule primaire (dp) de 15 à 100 nm, et une taille médiane d'aggrégat de 0,11 um à 10 μm.

7. Carbonate de calcium précipité selon l'une quelconque des revendications 1 à 6, dans lequel le carbonate de calcium précipité a une valeur de perte au séchage égale ou inférieure à 15 g/kg à 105 °C.

8. Processus de préparation de particules de carbonate de calcium précipitées se présentant au moins partiellement sous la forme de nanofibres ou de nanochaînes comme agglomérats, dans lequel les particules sont au moins partiellement recouvertes d'au moins un agent de revêtement, qui comprend une étape de mise en contact de particules de carbonate de calcium précipitées étant au moins partiellement sous la forme de nanofibres ou de nanochaînes comme agglomérats avec au moins un agent de revêtement, dans lequel les nanofibres ou les nanochaînes comme agglomérats sont en outre agrégés pour former des microcoques, dans lequel le processus comprend :

(a) la précipitation des particules de carbonate de calcium à partir d'une suspension comprenant de l'hydroxyde de calcium et de l'eau dans des conditions fournissant des particules de carbonate de calcium précipitées se présentant au moins partiellement sous la forme de nanofibres ou de nanochaînes comme agglomérats, la suspension comprenant en outre l'acide polyacrylique comme régulateur de cristallisation, dans lequel l'acide polyacrylique est ajouté avant la carbonatation et dans lequel l'acide polyacrylique est présent dans l'eau de chaux en une quantité de 0,1 à 1 % en poids, et
(b) la mise en contact des particules de carbonate de calcium obtenues à l'étape (a) avec l'au moins un agent de revêtement.

9. Procédé selon la revendication 8, dans lequel la précipitation des particules de carbonate de calcium à l'étape (a) est démarrée à une température de l'eau de chaux qui est comprise entre 10 et 30 °C.

10. Procédé selon la revendication 8 ou la revendication 9, dans lequel la température de l'eau de chaux au début de la carbonatation est comprise entre 12 et 20 °C.

11. Procédé selon les revendications 8 à 10, dans lequel à la fin de la carbonatation, une température comprise entre 15 et 70 °C, de préférence entre 55 et 65 °C est maintenue.

12. Utilisation de particules de carbonate de calcium précipitées selon l'une quelconque des revendications 1 à 7 comme additif, par exemple charge, dans les peintures, les plastisols, les mastics, le papier, les produits alimentaires, les produits nutritionnels, les produits pharmaceutiques, les encres, les laques et les polymères, notamment dans les plastisols.

13. Peinture, plastisol, mastic, papier, produit alimentaire, produit nutritionnel, produit pharmaceutique, encre, laque, polymère et compositions polymères, notamment plastisol, comprenant des particules de carbonate de calcium précipitées selon l'une quelconque des revendications 1 à 7.

14. Plastisol comprenant des particules de carbonate de calcium précipitées selon l'une quelconque des revendications 1 à 7.

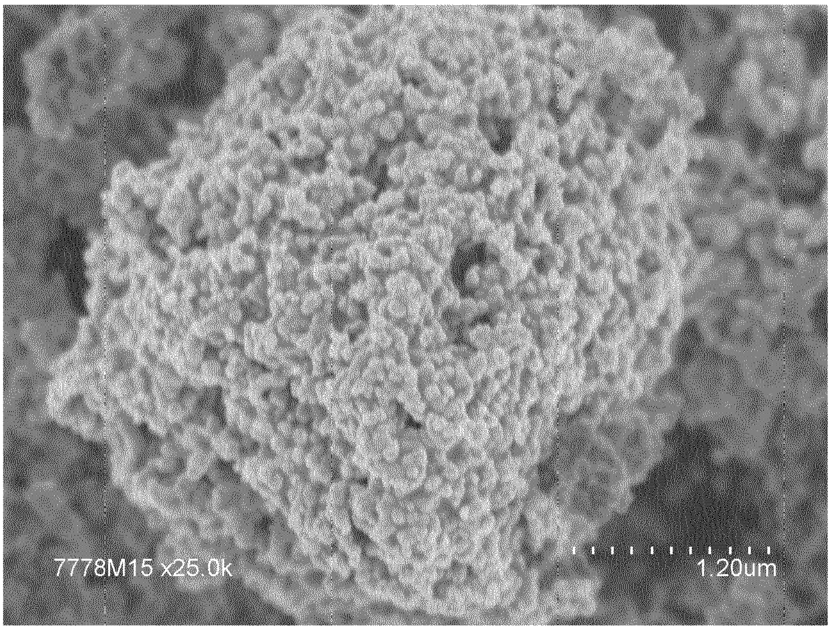


Fig. 1: SEM micrography of PCC2.

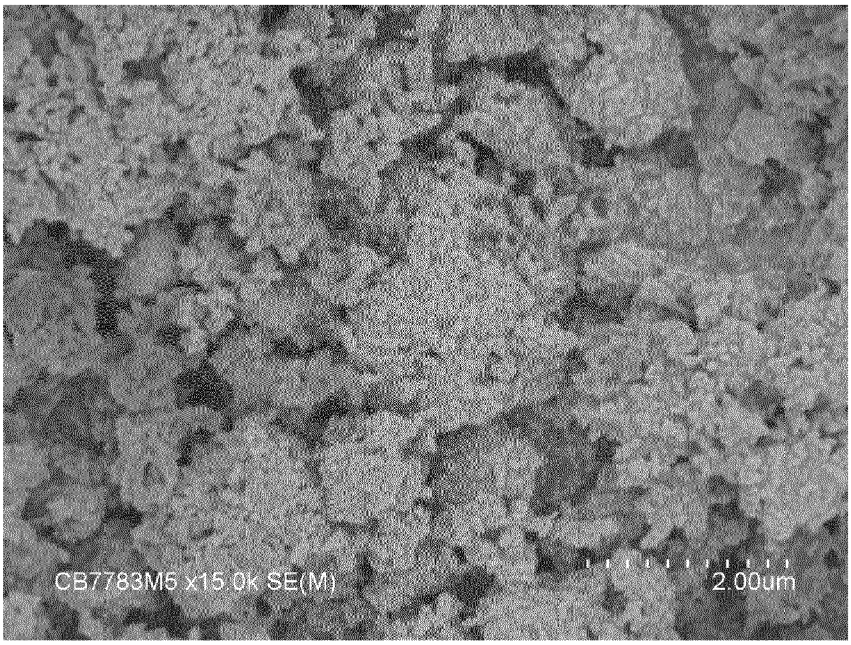


Fig. 2: SEM micrography of PCC3.

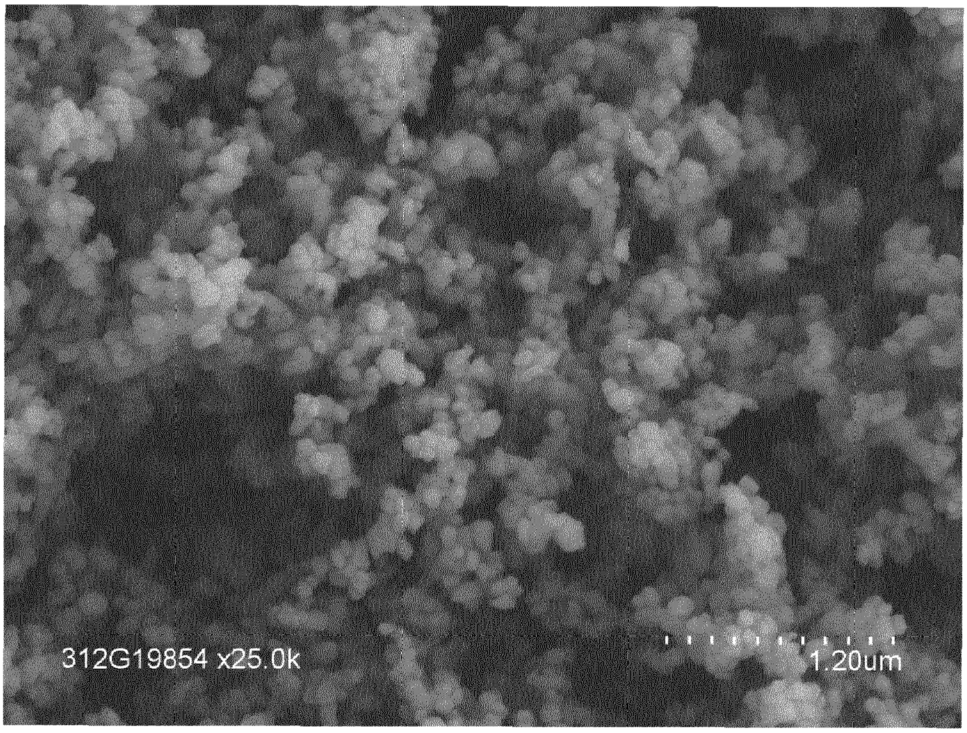

Fig. 3: SEM micrography of Socal ® 312.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013050495 A
- WO 2006051087 A